(19) Europäisches Patentamt
European Patent Office
Office européen des brevets

(11) Veröffentlichungsnummer: **0 052 201**
**B1**

# (12) EUROPÄISCHE PATENTSCHRIFT

(45) Veröffentlichungstag der Patentschrift: **30.05.84**

(51) Int. Cl.³: **C 07 D 317/30**, C 07 D 319/06, C 07 D 207/16

(21) Anmeldenummer: **81107464.0**

(22) Anmeldetag: **19.09.81**

(54) **Cyclische Acetale des Glutaminsäure-gamma-semialdehyds, Verfahren zu deren Herstellung und ihre Verwendung.**

(30) Priorität: **15.11.80 DE 3043159**

(43) Veröffentlichungstag der Anmeldung: **26.05.82 Patentblatt 82/21**

(45) Bekanntmachung des Hinweises auf die Patenterteilung: **30.05.84 Patentblatt 84/22**

(84) Benannte Vertragsstaaten: **BE CH DE FR GB LI NL**

(56) Entgegenhaltungen:
**DE - C - 800 574**
**US - A - 2 557 920**

**Chemical Abstracts Volume 91, 1979, Number 2113h**
**Methoden der organischen Chemie-Houben Weyl, 4 Auflage, Band XI/2 S. 371**

(73) Patentinhaber: **Degussa Aktiengesellschaft, Weissfrauenstrasse 9, D-6000 Frankfurt am Main 1 (DE)**

(72) Erfinder: **Drauz, Karlheinz, Dr. Dipl.-Chem, Flurstrasse 5, D-6463 Freigericht 1 (DE)**
Erfinder: **Kleemann, Axel, Dr. Dipl.-Chem., Greifenhagenstrasse 25, D-6450 Hanau 9 (DE)**
Erfinder: **Samson, Marc, Dr., Grünaustrasse 1, D-6450 Hanau 9 (DE)**

**EP 0 052 201 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung sind cyclische Acetale des Glutaminsäure-γ-semialdehyds der Formel

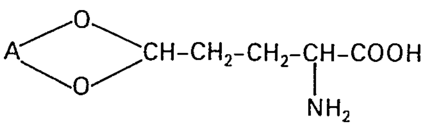

(I),

in der A eine unsubstituierte oder durch 1 bis 2 Methylgruppen substituierte Alkylengruppe mit 2 oder 3 Kohlenstoffatomen bedeutet, und ein Verfahren zu ihrer Herstellung.

Diese cyclischen Acetale des Glutaminsäure-γ-semialdehyds sind wertvolle Zwischenprodukte für die Herstellung von D,L-Prolin. Ein weiterer Gegenstand der Erfindung ist daher ihre Verwendung zur Herstellung von D,L-Prolin.

Aus der DE-C – 800 574 ist zwar bereits ein Verfahren bekannt, nach dem D,L-Prolin aus δ-Oxy-α-aminovaleriansäure in einer Ausbeute von 85% der Theorie hergestellt werden kann. Die bei dem bekannten Verfahren als Ausgangsmaterial dienende δ-Oxy-α-aminovaleriansäure ihrerseits ist aber nur sehr schwer zugänglich.

Die cyclischen Acetale des Glutaminsäure-γ-semialdehyds der allgemeinen Formel (I) können hergestellt werden nach einem Verfahren, welches dadurch gekennzeichnet ist, dass man

a) eine Verbindung der allgemeinen Formel

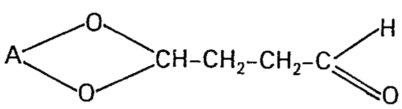

(II),

in der A wieder die bereits angegebene Bedeutung hat, in an sich bekannter Weise in wässriger oder wässrig-alkoholischer Lösung mit Cyanwasserstoff oder einer Cyanidionen liefernden Verbindung, Ammoniak oder einer Ammoniumionen liefernden Verbindung und Kohlendioxid oder einer Carbonationen liefernden Verbindung zur Reaktion bringt und b) das in Stufe a) erhaltene Reaktionsgemisch unter basischen Reaktionsbedingungen hydrolysiert.

Die beiden Reaktionsstufen des erfindungsgemässen Verfahrens verlaufen mit hohen Umsätzen.

Da auch die einzusetzenden Verbindungen der allgemeinen Formel (II) durch Hydroformylierung der entsprechenden 2-Vinyl-1,3-dioxolane bzw. 2-Vinyl-1,3-dioxane und letztere durch Acetalisierung von Acrolein mit den entsprechenden 1,2- bzw. 1,3-Glykolen leicht und in hohen Ausbeuten erhältlich sind, können die cyclischen Acetale des Glutaminsäure-γ-semialdehyds der allgemeinen Formel (I) kostengünstig hergestellt werden. Da die Verbindungen der allgemeinen Formel (I) dann ebenfalls leicht und mit hohen Ausbeuten in D,L-Prolin umgewandelt werden können, wird insgesamt ein neuer, von Acrolein ausgehender, vorteilhafter und kostengünstiger Weg zum D,L-Prolin eröffnet.

Beispiele für einzusetzende Verbindungen der allgemeinen Formel (II) sind 2-(2′-Formyläthyl)-1,3-dioxolan, 2-(2′-Formyläthyl)-4-methyl-1,3-dioxolan, 2-(2′-Formyläthyl)-4,5-dimethyl-1,3-dioxolan, 2-(2′-Formyläthyl)-1,3-dioxan, 2-(2′-Formyläthyl)-4-methyl-1,3-dioxan oder 2-(2′-Formyläthyl)-5,5-dimethyl-1,3-dioxan.

Die Verbindungen der allgemeinen Formel (II) werden in einer ersten Reaktionsstufe in der für die Bildung von Hydantoinen aus Aldehyden an sich bekannten Weise mit Cyanwasserstoff oder einer Cyanidionen liefernden Verbindung, wie Natriumcyanid oder Kaliumcyanid, mit Ammoniak oder einer Ammoniumionen liefernden Verbindung, wie Ammoniumhydroxid oder Ammoniumchlorid, und mit Kohlendioxyd oder einer Carbonationen liefernden Verbindung, wie Natrium- oder Kaliumbicarbonat, -carbonat oder -carbamat, umgesetzt. Es können auch Verbindungen eingesetzt werden, die gleichzeitig Cyanid- und Ammoniumionen liefern, wie Ammoniumcyanid, oder die gleichzeitig Ammonium- und Carbonationen liefern, wie Ammoniumcarbonat oder Ammoniumcarbamat.

Die Umsetzung in der ersten Reaktionsstufe erfolgt in Wasser oder in einem Gemisch aus Wasser und Methanol oder Äthanol. Sie kann in einem weiten Temperaturbereich vorgenommen werden. Bevorzugt wird eine Temperatur zwischen 30 und 90°C, weil in diesem Bereich eine zufriedenstellende Reaktionsgeschwindigkeit erreicht wird und der eventuell erforderliche Überdruck technisch kein Hindernis bildet.

Die Mengen der einzelnen Reaktionsteilnehmer können innerhalb weiter Grenzen variiert werden. Vorzugsweise werden je Mol Verbindung der allgemeinen Formel (II) 1 bis 1,5 Mol Cyanwasserstoff oder einer Cyanidionen liefernden Verbindung, 2 bis 15 Mol Ammoniak oder einer Ammoniumionen liefernden Verbindung und 1 bis 2 Mol Kohlendioxid oder einer Carbonationen liefernden Verbindung eingesetzt. Die Verbindungen der allgemeinen Formel (II) können gleichzeitig mit allen drei anderen Reaktionsteilnehmern umgesetzt werden. Ebenso ist es aber auch möglich, sie zunächst nur mit der Cyanidkomponente und anschliessend mit den beiden anderen Komponenten gleichzeitig, oder zunächst nur mit der Cyanidkomponente, dann nur mit der Ammoniumkomponente und dann erst mit der Kohlendioxid- bzw. Carbonatkomponente umzusetzen. Besonders vorteilhaft ist es, wenn die Verbindung der allgemeinen Formel (II) in Methanol oder Äthanol gelöst und diese Lösung langsam zu einer auf die gewünschte Reaktionstemperatur erwärmten wässrigen Lösung oder Suspension der anderen Reaktionsteilnehmer zudosiert wird. Zur Erzielung eines hohen Umsatzes ist eine angemessene Nachreaktionszeit von beispielsweise 5 Stunden nach dem Ende der Dosierung zu empfehlen.

Je nach den angewandten Reaktionsbedingungen enthält das Reaktionsgemisch nach der Durchführung der ersten Reaktionsstufe neben dem zu erwartenden Hydantoin der allgemeinen Formel

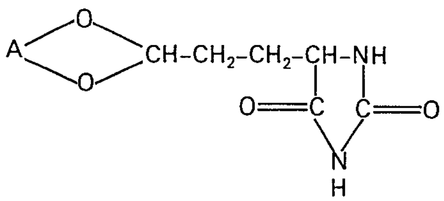
(III)

auch noch einen mehr oder weniger grossen Anteil an dem α-N-Carbamoyl-carbonsäureamid der allgemeinen Formel

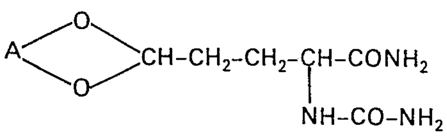
(IV)

wobei in den Formeln (III) und (IV) A wieder die oben bereits angegebene Bedeutung hat.

Eine Trennung der beiden Reaktionsprodukte ist jedoch nicht erforderlich, da sie beide bei der nachfolgenden Reaktionsstufe b) zu den cyclischen Acetalen des Glutaminsäure-γ-semialdehyds umgesetzt werden. Es kann aber zweckmässig sein, vor Durchführung der zweiten Reaktionsstufe b) die im rohen Reaktionsgemisch der ersten Reaktionsstufe a) enthaltenen Ammoniumsalze zu verkochen, den gegebenenfalls enthaltenen Alkohol abzudestillieren und das Reaktionsgemisch unter vermindertem Druck einzuengen.

In der Reaktionsstufe b) wird nun das in Stufe a) erhaltene Gemisch von Verbindungen der allgemeinen Formeln (III) und (IV) in der für die Bildung von α-Aminosäuren aus den entsprechenden substituierten Hydantoinen an sich bekannten Weise unter basischen Hydrolysebedingungen umgesetzt. Bevorzugt werden Alkali- oder Erdalkalimetallhydroxide oder Alkalimetallcarbonate in wässrigem Medium verwendet. Beipielsweise können mit gutem Erfolg NaOH, KOH, $Na_2CO_3$, $K_2CO_3$, $Ca(OH)_2$ oder $Ba(OH)_2$ eingesetzt werden. Die Reaktionstemperatur kann in weiten Grenzen zwischen 20°C und 200°C variiert werden. Bevorzugt werden Temperaturen zwischen 120°C und 170°C, da in diesem Bereich zufriedenstellende Reaktionsgeschwindigkeiten erreicht werden. Besonders bevorzugte Verseifungsbedingungen sind Temperaturen zwischen 130°C und 160°C, Reaktionszeiten von 0,5 bis 3 Stunden und ein Molverhältnis von Substrat zu Base von 1:2 bis 1:2,5.

Nach der Hydrolyse wird das Reaktionsgemisch neutralisiert und das gebildete Glutaminsäure-γ-semialdehydacetal der allgemeinen Formel (I) isoliert. Das kann beispielsweise durch Adsorption an einem stark sauren Ionenaustauscher geschehen.

Da die Verbindungen der allgemeinen Formel (I) im allgemeinen leicht in Wasser löslich sind, ist es aber unter Umständen vorteilhafter, bei der Hydrolyse als Base ein Erdalkalimetallhydroxid bzw. -oxid einzusetzen und die Neutralisierung des Reaktionsgemisches mit einem Neutralisierungsmittel vorzunehmen, welches mit der eingesetzten Base ein in Wasser schwerlösliches Salz bildet, insbesondere Kohlendioxid. Man erhält dann durch einfaches Abfiltrieren des ausgefallenen Salzes eine praktisch reine wässrige Lösung des Glutaminsäure-γ-semialdehyd-acetals, die unmittelbar zur Herstellung von Prolin eingesetzt werden kann. Sie kann aber natürlich auch, gegebenenfalls unter vermindertem Druck, soweit eingeengt werden, dass sich das Glutaminsäure-γ-semialdehyd-acetal in kristalliner Form abscheidet.

Um die Glutaminsäure-γ-semialdehyd-acetale der allgemeinen Formel (I) in D,L-Prolin umzuwandeln, werden sie bei einem pH zwischen 0 und 4, vorzugsweise zwischen 0,5 und 3, unter den Bedingungen einer katalytischen Hydrierung mit Wasserstoff umgesetzt. Der erforderliche pH kann durch eine anorganische Säure, wie Salzsäure, Schwefelsäure oder Phosphorsäure, oder durch eine organische Säure, wie Oxalsäure, Ameisensäure, Essigsäure, Benzolsulfonsäure oder p-Toluolsulfonsäure, eingestellt werden.

Als Lösungmittel für die hydrierende Behandlung dient Wasser oder ein Gemisch aus Wasser und einem damit mischbaren organischen Lösungsmittel, wie Methanol, Äthanol, Isopropylalkohol, n-Butanol, Tetrahydrofuran oder Dioxan.

Als Hydrierkatalysatoren werden im allgemeinen die Metalle der 8. Nebengruppe des Periodischen Systems oder geeignete Verbindungen derselben bevorzugt. Die Metalle können als solche, aber auch in an sich bekannter Weise auf geeignete Trägermaterialien aufgebracht verwendet werden. Besonders bevorzugte Katalysatoren sind Palladium und Platin und/oder deren Verbindungen. Beispiele für geeignete Katalysatoren sind feinverteiltes Palladiummetall, insbesondere als Palladium-Mohr, Palladiumbromid, -chlorid, -jodid, -cyanid, -arsenid, -nitrat, -oxid oder -sulfid oder Komplex-Salze, wie Tetrachloropalladate, Herachloropalladate, Tetraamin- oder Diaminpalladiumchlorid, sowie feinverteiltes Platinmetall, vor allem als Platin-Mohr, oder Platin-oxid.

Sollen Trägerkatalysatoren eingesetzt werden, so sind geeignete Trägermaterialien beispielsweise Aktivkohle, Bariumsulfate, Kieselgel, Aluminiumoxid oder Zeolithe.

Die Hydrierkatalysatoren werden zweckmässigerweise in einer Menge zwischen 0,01 und 50 Gewichtsprozent, vorzugsweise zwischen 0,1 und 10 Gewichtsprozent, berechnet als aktives Metall und bezogen auf das Gewicht des eingesetzten Glutaminsäure-γ-semialdehyd-acetals, angewandt. Die hydrierende Behandlung wird kontinuierlich oder diskontinuierlich in üblicher Weise

bei einer Temperatur zwischen 0 und 200°C, vorzugsweise zwischen 20 und 100°C, drucklos oder bei einem Wasserstoffdruck bis zu etwa 100 bar vorgenommen.

Nach der hydrierenden Behandlung wird der Katalysator durch Filtration aus dem Reaktionsgemisch abgetrennt und das gebildete D,L-Prolin in an sich bekannter Weise, z. B. mittels eines Ionenaustauschers, isoliert.

Durch die nachfolgenden Beispiele soll die Erfindung näher verdeutlicht werden. Sofern nicht anders angegeben, bedeuten die Prozentangaben Gewichtsprozente.

Beispiel 1:

Zu einer Suspension aus 48 g Ammoniumcarbonat, 5,1 g Blausäure und 110 ml wässrigem Ammoniak (25%ig) werden bei 35°C im Verlaufe einer Stunde 16,3 g 2-(2'-Formyl-äthyl)-1,3-dioxolan zugetropft, und es wird fünf Stunden bei 40°C nachgerührt. Anschliessend werden durch Temperaturerhöhung (bis 100°C Kopftemperatur) die Salze verkocht. Die verbleibende wässrige Lösung wird mit 18,5 g Calciumhydroxid versetzt und 2,5 Stunden auf 150°C erhitzt. Nach dem Abkühlen auf 80°C wird das Reaktionsgemisch mit festem Kohlendioxid neutralisiert und das ausgefallene Calciumcarbonat heiss abfiltriert. Das wässrige Filtrat wird im Vakuum eingeengt und der verbleibende Rückstand aus Wasser-Äthanol (Volumenverhältnis 1:9) umkristallisiert. Man erhält 19,1 g (87% der Theorie) Glutaminsäure-γ-semialdehydäthylenacetal (Zersetzungspunkt ≥ 230°C).

Elementaranalyse: $C_7H_{13}NO_4$

Berechnet: C 47,99% H 7,48% N 7,80%
Gefunden: C 48,21% H 7,55% N 7,7%

$^1H$-NMR-Spektrum ($D_2O$)

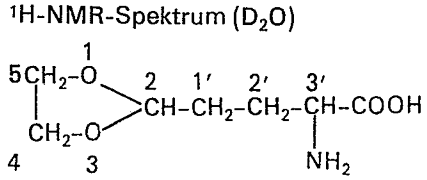

δ = 1,5 - 2,2 (m, 4H) : H-1', H-2'
δ = 3,75 (t, 1H) : H - 3'
δ = 3,7 - 4,2 (m, 4H) : H-4, H-5
δ = 4,95 (t, 1H) : H-2

Beispiel 2:

Zu einer Suspension aus 48 g Ammoniumcarbonat, 5,1 g Blausäure und 110 ml wässrigem Ammoniak (25%ig) werden bei 40°C im Verlaufe einer Stunde 16,3 g 2-(2'-Formyläthyl)-1,3-dioxolan zugetropft, und es wird fünf Stunden bei dieser Temperatur nachgerührt. Anschliessend werden durch Temperaturerhöhung (bis 100°C Kopftemperatur) die Salze verkocht. Die verbleibende wässrige Lösung wird mit 42 g Bariumhydroxid versetzt und 1 Stunde auf 160°C erhitzt. Nach dem Abkühlen auf 60° wird das Reaktionsgemisch mit Ammoniumcarbonat neutralisiert und das ausgefallene Bariumcarbonat heiss abfiltriert. Das Filtrat wird im Vakuum eingeengt und der Rückstand aus Wasser-Dioxan umkristallisiert. Man erhält 19,7 g (90% der Theorie) Glutaminsäure-γ-semialdehydäthylenacetal.

Beispiel 3:

Zu einer Suspension aus 42 g Ammoniumcarbonat, 6 g Blausäure und 120 ml wässrigem Ammoniak (25%ig) wird bei 50°C im Verlaufe einer Stunde eine Lösung von 20,7 g 2-(2'-Formyläthyl)-1,3-dioxan in 50 ml Methanol zugetropft, und es wird noch drei Stunden bei 50°C nachgerührt. Anschliessend werden bis zu einer Kopftermperatur von 100°C das Methanol abdestiliert und die Salze verkocht. Die verbleibende wässrige Lösung wird mit 21 g Calciumhydroxid versetzt und 2,5 Stunden auf 150°C erhitzt. Nach dem Abkühlen auf 80°C wird mit festem Kohlendioxid neutralisiert und das ausgefallene Calciumcarbonat heiss abfiltriert. Das wässrige Filtrat wird im Vakuum eingeengt und der verbleibende Rückstand aus Wasser-Äthanol (Volumenverhältnis: 1:9) umkristallisiert. Man erhält 23,3 g (86% der Theorie) Glutaminsäure-γ-semialdehydpropylen-1,3-acetal.

Elementaranalyse: $C_8H_{15}NO_4$

Berechnet: C 50,78% H 7,99% N 7,40%
Gefunden: C 51,31% H 8,15% N 7,35%

Beispiel 4:

Zu einer Suspension aus 20 g Ammoniumcarbonat, 4,8 ml Blausäure und 110 ml wässrigem Ammoniak (25%ig) wird bei 40°C im Verlaufe einer halben Stunde 17,2 g 2-(2'-Formyläthyl)-5,5-dimethyl-1,3-dioxan, gelöst in 50 ml Methanol, zugetropft, wonach die Temperatur noch 5 Stunden lang auf 40°C gehalten wird. Anschliessend werden das Methanol abdestilliert und durch weiteres Erhitzen auf 100°C die Ammoniumsalze verkocht. Die verbleibende wässrige Suspension wird mit 15 g Calciumhydroxid versetzt und 2 Stunden auf 160°C erhitzt. Nach dem Abkühlen auf 80°C wird das Reaktionsgemisch mit festem Kohlendioxid neutralisiert und heiss filtriert. Das Filtrat wird im Vakuum eingeengt und der Rückstand aus Wasser-Äthanol (Volumenverhältnis: 1:9) umkristallisiert. Man erhält 18,7 g (86% der Theorie) Glutaminsäure-γ-semialdehyd-2,2-dimethylpropylen-1,3-acetal.

Elementaranalyse: $C_{10}H_{19}NO_4$

Berechnet: C 55,28% H 8,81% N 6,45%
Gefunden: C 55,40% H 8,92% N 6,40%

$^1H$-NMR-Spektrum ($D_2O$)

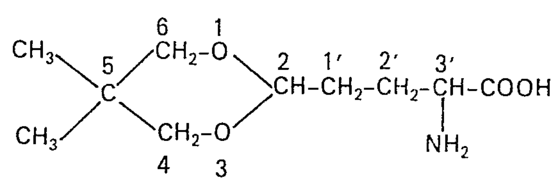

δ = 0,75 (s,3H) : 5-$CH_3$
δ = 1,15 (s,3H) : 5-$CH_3$
δ = 1,5–2,2 (m,4H) : H-1′, H-2′
δ = 3,65 (q, 4H) : H-4, H-6
δ = 3,75 (t, 1H) : H-3′
δ = 4,65 (t, 1H) : H-2

Beispiel 5:
2,0 g Glutaminsäure-γ-semialdehydäthylenacetal werden in 15 ml 0,5 N Salzsäure bei Raumtemperatur gelöst, 1,0 g Pd/C (10% Pd) zugegeben und mit Wasserstoff bei 1 bar bis zur berechneten Aufnahmemenge (250 ml) hydriert. Nach Abtrennen des Katalysators und Einengen des Filtrats erhält man 80,9% dünnschichtchromatographisch reines D,L-Prolin in Form des Hydrochlorids.

Beispiel 6:
6,0 g Glutaminsäure-γ-semialdehydäthylenacetal werden in 50 ml 0,25 N Salzsäure bei Raumtemperatur gelöst, 2,5 g Pd/C (10% Pd) zugegeben und bei 25°C in einem Autoklaven bei 20 bar $H_2$-Druck bis zum vollständigen Umsatz hydriert.
Nach Aufarbeitung erhält man 85,2% dünnschichtchromatographisch reines D,L-Prolin in Form des Hydrochlorids.

Beispiel 7:
10,0 g Glutaminsäure-γ-semialdehyäthylenacetal werden gemäss Beispiel 6 bei 50°C hydriert. Nach der Aufarbeitung erhält man 83,7% dünnschichtchromatographisch reines D,L-Prolin in Form des Hydrochlorids.

Beispiel 8:
5,0 g Glutaminsäure-γ-semialdehydäthylenacetal werden in 50 ml 0,1 N Schwefelsäure gemäss Beispiel 5 hydriert. Man erhält 84,2% dünnschichtchromatographisch reines D,L-Prolin als Sulfat.

Beispiel 9:
6,0 g Glutaminsäure-γ-semialdehydäthylenacetal werden in 50 ml 90%iger wässriger Ameisensäure gemäss Beispiel 6 hydriert. Die Aufarbeitung liefert 83,0% freies D,L-Prolin.

Beispiel 10:
4,0 g Glutaminsäure-γ-semialdehydäthylenacetal werden gemäss Beispiel 5 hydriert. Die salzsaure Lösung wird eingeengt, der Rückstand in 15 ml Wasser aufgenommen und über einen schwach basischen Ionenaustauscher gegeben. Das Prolin-haltige Eluat wird bis zur Trockene eingeengt. Man erhält 2,23 g von anderen Aminosäuren freies D,L-Prolin (84,8% der Theorie) mit Schmelzpunkt 208° – 213°C (Zers.).

Beispiel 11:
2,0 g Glutaminsäure-γ-semialdehydäthylenacetal werden mit 0,3 g Platinoxid gemäss Beispiel 6 hydriert.
Man erhält nach Aufarbeitung 81% dünnschichtchromatographisch reines D,L-Prolin in Form des Hydrochlorids.

Beispiel 12:
2,0 g Glutaminsäure-γ-semialdehyd-2,2-dimethylpropylen-1,3-acetal werden mit 1,0 g Pd/C (10%Pd) und 15 ml 0,1 N Salzsäure versetzt und in einem Autoklaven bei 50 bar und 50°C bis zum vollständigen Umsatz hydriert. Die Aufarbeitung ergibt 81,5% dünnschichtchromatographisch reines D,L-Prolin in Form des Hydrochlorids.

Beispiel 13:
18,9 g Glutaminsäure-γ-semialdehydpropylen-1,3-acetal werden in 150 ml 0,1 N Salzsäure gelöst, 5 g Pd/C (5% Pd) zugegeben und mit Wasserstoff bei Raumtemperatur unter Normaldruck bis zum vollständigen Umsatz hydriert. Nach Aufarbeitung erhält man 13,0 g (86,0%) D,L-Prolinhydrochlorid, dünnschichtchromatographisch rein.
Daraus werden nach Dehydrohalogenierung mit einem schwach basischen Ionenaustauscher 9,78 g (85,0%) D,L-Prolin erhalten. Schmelzpunkt: 207–212°C (Zers.).

**Patentansprüche:**

1. Cyclische Acetale des Glutaminsäure-γ-semialdehyds der allgemeinen Formel

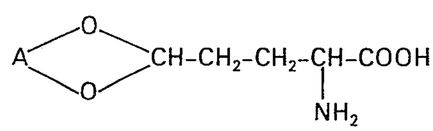
(I),

in der A eine unsubstituierte oder durch 1 bis 2 Methylgruppen substituierte Alkylengruppe mit 2 oder 3 Kohlenstoffatomen bedeutet.

2. Verfahren zur Herstellung der cyclischen Acetale des Glutaminsäure-γ-semialdehyds der allgemeinen Formel (I) gemäss Anspruch 1, dadurch gekennzeichnet, dass man

a) eine Verbindung der allgemeinen Formel

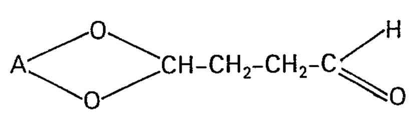
(II),

in der A wieder die bereits angegebene Bedeutung hat, in an sich bekannter Weise in wässriger oder wässrig-alkoholischer Lösung mit Cyanwasserstoff oder einer Cyanidionen liefernden Verbindung, Ammoniak oder einer Ammoniumionen liefernden Verbindung und Kohlendioxid oder einer Carbonationen liefernden Verbindung zur Reaktion bringt und

b) das in Stufe a) erhaltene Reaktionsgemisch unter basischen Reaktionsbedingungen hydrolysiert.

3. Verfahren nach Anspruch 2, dadurch gekennzeichnet, das man in der Stufe a) den Cyanwasserstoff oder die Cyanidionen liefernde Verbindung in einer Menge zwischen 1 und 1,5 Mol, den Ammoniak oder die Ammoniumionen liefernde Verbindung in einer Menge zwischen 2 und 15 Mol und das Kohlendioxid oder die Carbonationen liefernde Verbindung in einer Menge zwischen 1 und 2 Mol, jeweils pro Mol eingesetzter Verbindung der allgemeinen Formel (II), einsetzt.

4. Verfahren nach Anspruch 2 oder 3, dadurch gekennzeichnet, dass man die Stufe a) bei einer Temperatur zwischen 20 und 90°C durchführt.

5. Verfahren nach einem der Ansprüche 2 bis 4, dadurch gekennzeichnet, dass man die Stufe b) bei einer Temperatur zwischen 120 und 170°C durchführt.

6. Verwendung der cyclischen Acetale des Glutaminsäure-γ-semialdehyds der allgemeinen Formel (I) gemäss Anspruch 1 zur Herstellung von D,L-Prolin.

**Claims:**

1. Cyclic acetals of glutamic acid-γ-semialdehyde corresponding to the general formula

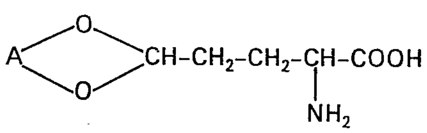 (I),

wherein

A represents an alkylene group having 2 or 3 carbon atoms which may be unsubstituted or substituted by from 1 to 2 methyl groups.

2. A process for the production of cyclic acetals of glutamic acid-γ-semialdehyde corresponding to the general formula (I) according to claim 1, characterised in that

a) a compound corresponding to the general formula

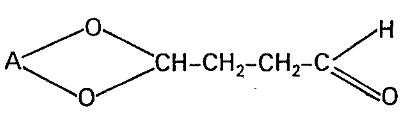 (II),

wherein

A is as defined above, is reacted in known manner in aqueous or aqueous-alcoholic solution with hydrogen cyanide or a cyanide ion-producing compound, ammonia or an ammonium ion-producing compound and carbon dioxide or a carbonate ion-producing compound, and

b) the reaction mixture which is obtained in stage a) is hydrolysed under basic reaction conditions.

3. a process according to claim 2, characterised in that in stage a), the hydrogen cyanide or the cyanide ion-producing compound is used in a quantity of from 1 to 1.5 mols, the ammonia or the ammonium ionproducing compound is used in a quantity of from 2 to 15 mols and the carbon dioxide or the carbonate ionproducing compond is used in a quantity of from 1 to 2 mols, in each case per mol of the compound corresponding to the general formula (II) which is used.

4. A process according to claim 2 or 3, characterised in that stage a) is carried out at a temperature of from 30 to 90° C.

5. A process according to one of claims 2 to 4, characterised in that stage b) is carried out at a temperature of from 120 to 170° C.

6. The use of the cyclic acetals of glutamic acid-γ-semialdehyde corresponding to the general formula (I) according to claim 1 for the production of D,L-proline.

**Revendications**

1) Acétals cycliques de l'acide γ-semialdéhyde-glutamique de formule générale:

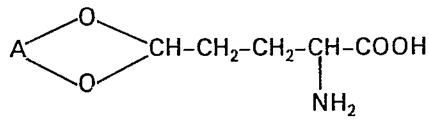 (I),

où A représente un groupe alcoylène non substitué ou substitué avec 1 à 2 groupes méthyle, et comportant 2 ou 3 atomes de carbone.

2) Procédé de préparation des acétals cycliques de l'acide γ-semialdéhyde-glutamique de formule générale (I), selon la revendication 1, caractérisé en ce que:

a) l'on fait réagir un composé de formule générale:

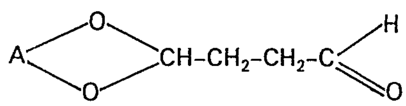 (II),

dans laquelle A a la même signification que ci-dessus, de manière connue en soi, en solution aqueuse ou hydroalcoolique, avec de l'acide cyanhydrique ou un composé libérant des ions cyanure, de l'ammoniac ou un composé libérant des ions ammonium, et du dioxyde de carbone ou un composé libérant des ions carbonate, et

b) l'on hydrolyse le mélange réactionnel obtenu dans l'étape a) dans des conditions réactionnelles basiques.

3) Procédé selon la revendication 2, caractérisé en ce que dans l'étape a) on met en œuvre l'acide cyanhydrique ou le composé libérant des ions cyanure en une proportion comprise entre 1 et 1,5 mole, l'ammoniac ou le composé libérant des ions ammonium, en proportion comprise entre 2 et 15 moles et le dioxyde de carbone ou le

composé libérant des ions carbonate en proportion comprise entre 1 et 2 moles, chaque fois par mole de composé de formule générale (II) mise en œuvre.

4) Procédé selon l'une des revendications 2 et 3, caractérisé en ce que l'étape a) est réalisée à une température comprise entre 30 et 90°C.

5) Procédé selon l'une quelconque des revendications 2 à 4, caractérisé en ce que l'étape b) est réalisée à une température comprise entre 120 et 170°C.

6) Utilisation des acétals cycliques de l'acide γ-semialdéhyde-glutamique de formule générale (I) selon la revendication 1, pour la préparation de D,L-proline.